# EUROPEAN PATENT SPECIFICATION

(11) **EP 3 047 741 B1**
(45) Date of publication and mention of the grant of the patent: **16.05.2018**
(21) Application number: 15151918.8
(22) Date of filing: 21.01.2015
(51) Int. Cl.: A24F 47/00, H01M 2/10

(54) **Electronic smoking device**
Elektronische Rauchvorrichtung
Dispositif à fumer électronique

(43) Date of publication of application: 27.07.2016
(73) Proprietor: Fontem Holdings 1 B.V., 1083 HN Amsterdam (NL)
(72) Inventor: Borkovec, Vaclav, 22761 Hamburg (DE); Wedekind, Ralf, 79254 Oberried (DE); Fröchte, Heinz, 78658 Zimmern (DE)
(74) Representative: Gulde & Partner

(56) References cited:
- WO-A1-2015/006270
- US-A1- 2012 225 340
- US-A1- 2014 196 716
- US-B1- 6 228 517

## Description

### FIELD OF INVENTION

The present invention relates generally to electronic smoking devices and in particular electronic cigarettes.

### BACKGROUND OF THE INVENTION

An electronic smoking device, such as an electronic cigarette (e-cigarette), usually has a housing accommodating an electric power source (e.g. a single use battery or a rechargeable battery), and an electrically operable atomizer. The atomizer vaporizes or atomizes liquid supplied from a reservoir and provides vaporized or atomized liquid as an aerosol. Control electronics control the activation of the atomizer. In many electronic cigarettes, an airflow sensor is provided within the electronic smoking device which detects a user puffing on the device (e.g., by sensing an under-pressure or an air flow pattern through the device). The airflow sensor indicates or signals the puff to the control electronics. Alternatively, a button may be used to switch on the electronic smoking device to generate a puff of vapor. When a puff is detected, the control electronics supplies electrical power to the atomizer thereby creating vaporized liquid as an aerosol. Electronic smoking devices are disclosed in e.g. US 2014/196716 A1 and US 2012/225340 A1.

Some rechargeable batteries tend to expand over lifetime due to an accumulation of gases that are released when the battery is under load, which presents engineering challenges in the design of electronic smoking devices

### SUMMARY OF THE INVENTION

In accordance with one aspect of the present invention there is provided an electronic cigarette which includes a housing with a battery compartment. The electronic cigarette further includes a rechargeable battery arranged inside the battery compartment. The battery comprises two end surfaces opposed along a central axis and a peripheral surface connecting the end surfaces. Battery terminals are provided on at least one of the front sides. The electronic cigarette also includes a holding member biased against the peripheral surface of the battery and adapted to secure the battery inside the battery compartment regardless of its change of size. The holding member is adapted to press the battery against a wall of the battery compartment opposite to the holding member.

The characteristics, features and advantages of this invention and the manner in which they are obtained as described above, will become more apparent and be more clearly understood in connection with the following description of exemplary embodiments, which are explained with reference to the accompanying drawings.

### BRIEF DESCRIPTION OF THE DRAWINGS

In the drawings, same element numbers indicate same elements in each of the views:
Figure 1 is a schematic cross-sectional illustration of an exemplary e-cigarette;
Figure 2 is a schematic cross-sectional illustration of an exemplary battery compartment accommodating an unused battery.
Figure 3 is a schematic cross-sectional illustration of an exemplary battery compartment accommodating a used rechargeable battery.
Figure 4 is a schematic longitudinal-sectional illustration of a battery compartment with an exemplary holding member.
Figure 5 is a schematic longitudinal-sectional illustration of a battery compartment with a further exemplary holding member.
Figure 6 is a schematic longitudinal-sectional illustration of a battery compartment with an even further exemplary holding member.
Figure 7 is a schematic longitudinal-sectional illustration of a battery compartment with an even further exemplary holding member.
Figure 8 is a schematic cross-sectional view of the battery compartment with an even further exemplary holding member shown in Figure 7.
Figure 9 is a schematic cross-sectional illustration of an exemplary rectangular battery compartment accommodating a rectangular rechargeable battery.

### DESCRIPTION OF THE PREFERRED EMBODIMENTS

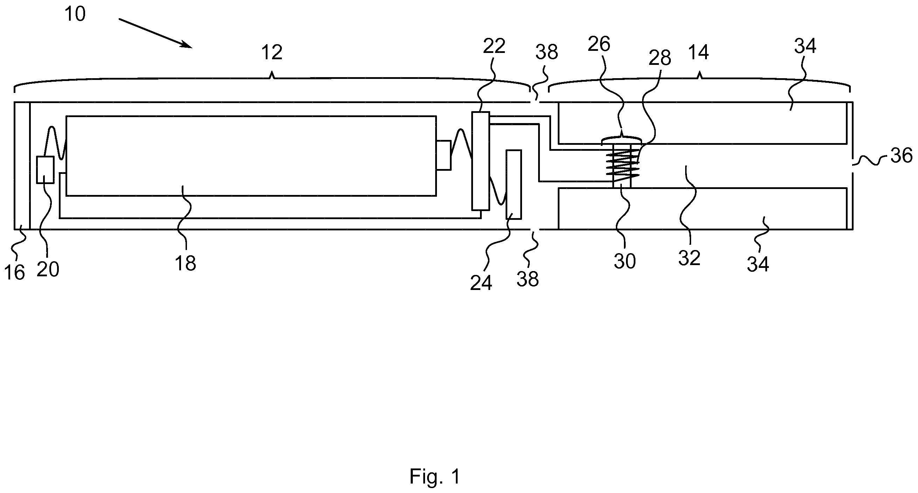

Prior to describing an embodiment of the present invention, the basic structure of an e-cigarette will first be described with reference to Figure 1 which is a schematic cross-sectional illustration of an exemplary e-cigarette.

As is shown in Figure 1, an e-cigarette 10 ordinarily comprises a cylindrical housing having a main body 12 and a mouthpiece portion 14. Together the main body 12 and the mouthpiece portion 14 form a cylindrical tube which is approximately the same size and shape as a conventional cigarette, typically about 100 mm with a 7.5 mm diameter, although lengths may range from 70 to 150 or 180 mm, and diameters from 5 to 20 mm. The main body 12 and mouthpiece 14 are typically made of steel or hardwearing plastic and act to provide a housing to contain the operative elements of the e-cigarette 10. The main body 12 and a mouthpiece portion 14 may be configured to fit together by means of a friction push fit, a snap fit, or a bayonet attachment, magnetic fit, or screwthreads. Alternatively in some embodiments a screw fit may be provided enabling the main body 12 and mouthpiece portion 14 to be attached to one another. Alternatively in some e-cigarettes the main body 12 and mouthpiece portion 14 may be parts of a single integrally formed tube.

An end cap 16 is provided at the end of the main body 12 remote from the mouthpiece portion 14 enclosing that end of the main body 12. The end cap 16 may be translucent plastic or other translucent material to allow an LED positioned near the end cap to emit light through the end cap. The end cap can be made of metal or other materials that do not allow light to pass. An air inlet may be provided in the end cap, at the edge of the inlet next to the cylindrical hollow tube, anywhere along the length of the cylindrical hollow tube, or at the connection of the main body 12 and the mouthpiece portion 14.

A battery 18 is provided within the central cavity enclosed by the main body 12. Also optionally contained within the central cavity defined by the main body 12 are a light emitting diode (LED) 20, control electronics 22 and an airflow sensor 24. The battery 18 is electrically connected to the LED 20 and the control electronics 22 and the airflow sensor 24 is connected to the control electronics 22. In this example the LED 20 is provided at one end of the main body 12, adjacent to the end cap 16 and the control electronics 22 and airflow sensor 24 are provided in the central cavity at the other end of the battery 18 adjacent the mouth piece portion 14.

The airflow sensor 24 acts as a puff detector, detecting a user puffing or sucking on the mouthpiece portion 14 of the e-cigarette 10. The airflow sensor 24 can be any suitable sensor for detecting changes in airflow or air pressure such a microphone switch including a deformable membrane which is caused to move by variations in air pressure. Alternatively the sensor may be a Hall element or an electro-mechanical sensor.

The control electronics 22 are also connected to an atomizer 26 which in this illustrative example comprises a heating coil 28 which is wrapped around a wick 30 which extends across a central passage 32 provided in the mouthpiece portion 14 of the e-cigarette 10. The dimensions of the central passage 32, the wick 30 and the heating coil 28 are such that the wick 30 and heating coil 28 do not completely block the central passage 32 but rather an air gap is provided at either side of the heating coil 28 enabling air to flow past the heating coil 28 and the wick 30.

The central passage 32 is surrounded by a cylindrical liquid store 34 with the ends of the wick 30 abutting or extending into the liquid store 34. The wick 30 comprises a porous material such as a bundle of fibreglass fibres such that liquid present in the liquid store 34 is drawn by capillary action from the ends of the wick 30 towards the central portion of the wick 30 encircled by the heating coil 28. The coil may be positioned anywhere along the liquid store 34 and may be positioned transverse or parallel to the liquid store.

In some embodiments the liquid store 34 will comprise wadding soaked in liquid which encircles the central passage 32 with the ends of the wick 30 abutting the wadding. In other embodiments the liquid store 34 may comprise a toroidal cavity arranged to be filled with liquid to be vaporized with the toroidal cavity enclosed by walls and with the ends of the wick 30 extending into the toroidal cavity.

An air inhalation port 36 is provided at the end of the mouthpiece portion 14 remote from main body 12 of the e-cigarette 10. One or more air inlets 38 are provided in the housing, optionally at the intersection between the main body 12 and the mouthpiece portion 14. The air inlets 38 may be adjacent the airflow sensor 24 with the central passage 32 within the mouthpiece portion 14 of the e-cigarette 10 extending from adjacent the air inlets 38 past the wick 30 and heating coil 28 to the air inhalation port 36. The inhalation port 36 may be formed from the cylindrical hollow tube of the main body 12 or it may be formed in an end cap.

In use, a user sucks on the mouthpiece portion 14 of the e-cigarette 10. This causes air to be drawn into the e-cigarette 10 via the air inlets 38 and to be drawn up via the central passage 32 towards the air inhalation port 36. The change in air pressure which arises is detected by the airflow sensor 24 which generates an electrical signal that is passed to the control electronics 22. In response to the signal, the control electronics 22 then proceed to activate the heating coil 28 which causes liquid present in the wick 30 to be vaporized creating an aerosol (which may comprise gaseous and liquid components) within the central passage 32. As the user continues to suck on the mouthpiece portion 14 of the e-cigarette 10, this aerosol is drawn up the central passage 32 and inhaled by the user sucking on the e-cigarette 10. At the same time the control electronics 22 also activate the LED 20 causing the LED 20 to light up which is visible via the translucent end cap 16 mimicking the appearance of a glowing ember at the end of a conventional cigarette. As liquid present in the wick 30 is converted into an aerosol more liquid is drawn into the wick 30 from the liquid store 34 by capillary action and thus is available to be converted into an aerosol through subsequent activation of the heating coil 28.

In some e-cigarettes, the e-cigarette 10 is intended to be disposable and the electric power in the battery 18 is intended to be sufficient to vaporize the liquid contained within the liquid store 34 after which the e-cigarette 10 is thrown away. In other embodiments the battery 18 is rechargeable and a means is provided to replenish the liquid supply. In the cases where the liquid store 34 is a toroidal cavity, this may be achieved by providing a refill port and refilling the cavity with liquid via the refill port. In other embodiments the mouthpiece portion 14 of the e-cigarette 10 is detachable from the main body 12 and a new mouthpiece portion 14 can be fitted with a new liquid store 34 thereby replenishing the supply of liquid. In some cases, replacing the liquid store 34 may involve replacement of the heating coil 28 and the wick 30 along with the replacement of the liquid store 34.

In some cases the new liquid store 34 may be in the form of a cartridge. In some such embodiments the cartridge may be provided with a central passage 32 through which a user inhales aerosol generated by the e-cigarette. In other embodiments, rather than inhaling aerosol via a central passage 32, the cartridge may be such to block the central portion of the e-cigarette 10 and generated aerosol may be directed around the exterior of the cartridge 32 to an air inhalation port 36 for inhalation.

It will also be appreciated that although the above description is illustrative of the structure and function of a typical e-cigarette 10, variations also exist. Thus for example in some e-cigarettes the LED 20 is omitted. In some e-cigarettes, the airflow sensor 24 may be placed adjacent the end cap 16 of the e-cigarette rather than in the middle of the e-cigarette as illustrated. Similarly, in some e-cigarettes, the air inlets 36 may be placed at the distal end of the main body 16 of the e-cigarette 10 remote from the mouthpiece portion 14. In some e-cigarettes the airflow sensor 24 is omitted and instead a button is provided which enables a user to activate the e-cigarette manually rather than in response to the detection of a change in air flow or air pressure. Also in some e-cigarettes the constitution of the atomizer may be changed. Thus for example rather than being constituted by a wick 28 surrounded by a heating coil 26 other configurations may be used such as providing a heating coil in the interior of a porous body and generating an aerosol by heating air and passing the air through the porous body. Further in some embodiments rather than generating an aerosol through heating liquid within a wick 28 an aerosol might be generated using a piezoelectric atomizer to create an aerosol for inhalation either in combination or in the absence of a heater.

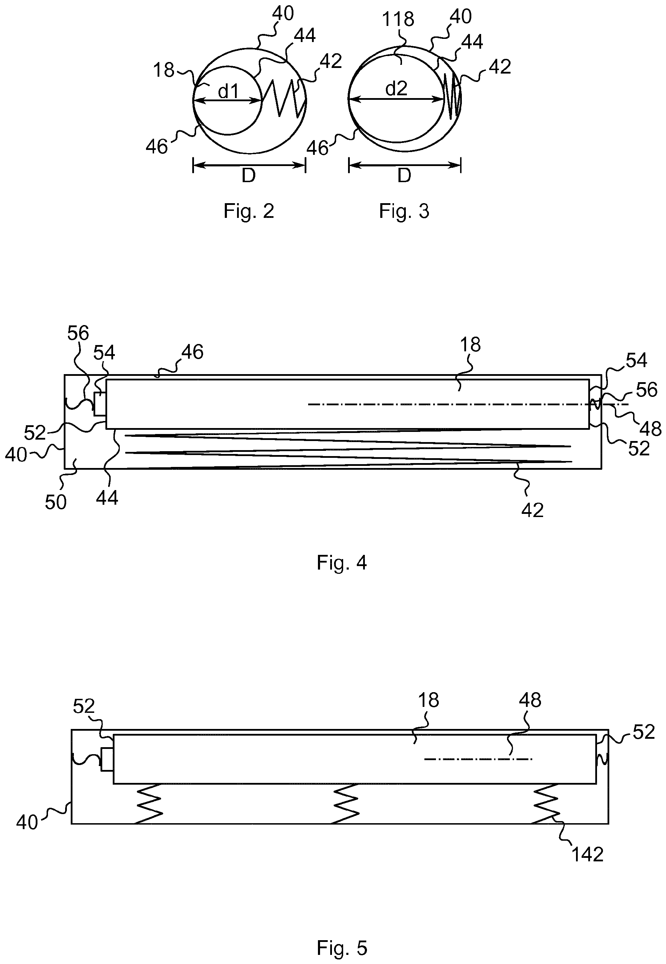

Referring to Figures 2 and 3 a principle of the present invention is disclosed. The battery 18, preferably a rechargeable or secondary battery, is located inside a battery compartment 40. The battery compartment 40 is arranged inside the main body 12. In case of a one piece cigarette the battery compartment 40 is arranged inside the tube constituted by the main body 12 and the mouthpiece portion 14. The battery compartment 40 may enclose the battery completely or partly. In the first case the battery compartment 40 may include an opening mechanism like a door or a lid so as to insert and remove the battery 40 from the battery compartment.

In Figure 2 a cross section of the battery compartment 40 is shown. Inside the battery compartment 40 the rechargeable battery 18 is arranged. The rechargeable battery 18 is an unused or new battery. This implies that battery 18 has a diameter d1 as of a time of manufacturing. The battery compartment 40 has a diameter D which is larger than the diameter d1 of the battery 18. In order to hold or immobilize the battery 18 inside the battery compartment 40 a holding member 42 is provided. The holding member 42 is biased against a peripheral surface 44 of the battery 18. The holding member 42 presses against the peripheral surface 44 of the battery 18 so that the battery 18 is pressed against a wall 46 of the battery compartment 40 or against another part. The holding member 42 is adapted to fit the battery 18 to or against the wall 46 of the battery compartment 40. The wall 46 of the battery compartment 40 is located at the opposite side of the holding member 42. Such arrangement fixes or secures the battery 18 regardless of its size or its change of size. The flexible biased holding member 42 compensates for size changes of the battery 18, e.g. due to increased load cycles and keeps the battery 18 still fixed. Advantageously, undesired movement of the battery 18 is prevented.

In Figure 3 the same battery compartment 40 is shown. Here, a used battery 118 is arranged inside the battery compartment 40. Battery 118 may have experienced a maximal size expansion due to charge/discharge cycles. Rechargeable batteries like for example lithium batteries expand during usage due to the accumulation of gases that are released when the battery is under load. This expansion may be in the range of 5 to 10 percent of the diameter over 3 to 500 charge/discharge cycles. Accordingly battery 118 has a diameter d2 which is larger than diameter d1 of the unused battery 18. Diameter d2 is still smaller than diameter D of the battery compartment 40. The size or diameter D of the battery compartment 40 may be designed or adapted to accommodate a battery 118 with maximal size expansion due to charge/discharge cycles. In other words, the diameter D of the battery compartment 40 is 5 percent, preferably 10 percent, larger than the diameter d1 of an unused battery 18 which is intended to be used inside the battery compartment 40 of the e-cigarette 10. While a cylindrical battery has been described a rectangular battery is encompassed as well. Figures 2 and 3 are not necessarily drawn in scale. Especially the increase from diameter d1 to diameter d2 is exaggerated for better perception.

In Figure 3 the holding member 42 presses or lines battery 118 against the wall 46 of the battery compartment 40. Due to the increase of the battery's 118 diameter, the holding member 42, here in form of a spring, is shown in a more compressed state than in Figure 2. Still, the holding member 42 is biased or pressed against the peripheral surface 44 of the battery 118 and immobilizes the battery 118 inside the battery compartment 40. The flexibility or elasticity of the holding member 42 covers the possible expansion of the battery 18. Hence, the holding member 42 equalizes or adapts for the diameter change of the battery 18 and keeps the battery fixed at all time regardless of its diameter. The battery 18 is held immobile while allowing for its expansion to take place without danger of the battery exploding or damaging its housing or the e-cigarette 10. As one example, with a standard R6 battery having a weight of about 25 grams, the holding member or spring 42 may have a spring constant and a preload which exerts a force of e.g. 0.5 to 2.0 N on the battery. Typical spring constants may be for example 0.4 N/mm to 2.0 N/mm. In general, the holding member 42 is designed to exert a force on the battery equal to about 2-10 times the weight of the battery, so that the battery is prevented from moving during ordinary use. The inner wall of the battery compartment 40 that the battery 118 rests against may also have features to help hold the battery in place, such as grooves, wedges or wheel chocks.

In order to allow expansion for the battery 18 inside the battery compartment 40 the diameter D of the battery compartment 40 is oversized in view of an unused battery 18 having a diameter d1. Without the holding member 42 an unused battery 18 would move inside the battery compartment 40 or hit walls of the battery compartment 40. The holding member 42 holds or immobilizes the battery 18 regardless of its diameter. Connections of the battery 18, especially soldered connections are relieved from stress which is imposed by a migrating battery. Further, dislodgement of positioning of components connected to the battery is also prevented. The user's experience is increased as no noise or momentum incurred by a lose battery occurs.

In some electronic cigarettes a dedicated battery compartment may be omitted. In such a case a part of the main body 12 functions as the battery compartment. The shape of the battery compartment 40 corresponds at least roughly to the shape of the battery 18. In other cases such as for especially designed batteries the shape of the battery 18 is adapted to the shape of the battery compartment 40. Here, the battery compartment 40 has a cylindrical shape. The battery compartment 40 may comprise or consist of plastic material.

Figure 4 shows a longitudinal sectional view of the battery compartment 40 including battery 18 in case the battery compartment 40 fully encloses battery 18. In case that battery compartment 40 surrounds battery 18 partly. Figure 4 shows a top or side view of the battery compartment 40 including battery 18.

The holding member 42 presses or abuts battery 18 against a wall 46 of the battery compartment 40. Holding member 42 is biased in a direction inside an inner space or cavity 50 of the battery compartment 40. More precisely, the holding member 42 exerts a force against the peripheral surface 44 of the battery 18 preferably in a direction perpendicular to a central axis 48 of the battery 18.

The battery 18 has two opposed end surfaces 52 having a diameter d1 as shown in Figure 2 in case of a new unused battery 18 or a diameter d2 as shown in Figure 3 for an expanded used battery 118. The two end surfaces 52 are opposed along the central axis 48 and are connected by the peripheral surface 44. Battery terminals 54 for electrically connecting the battery 18 are arranged at the end surfaces 52. Depicted is a configuration of a single battery terminal 52 arranged at one front side 52. Alternatively, it is possible to position both battery terminals 54 at or on one front side 52.

Electrical connection elements 56 like a cable, a metal clamp or a metal spring are permanently or removeably attached to the terminals 54 of the battery 18. The electrical connection elements 56 are in electrical connection to the control electronics 22, the LED 20 and/or the airflow sensor 24. The electrical connection elements 56 are adapted for allowing expansion and therefore movement of the battery 18 while keeping the electrical connection at every time. The electrical connection element 56 can be adapted accordingly for example by increasing the length of a cable, increasing the length and/or size of a metallic spring or contact. The electrical connection element 56 is not adapted to take the function of the holding member 42. Preferably, the function of holding the battery 18 regardless of its size change and the function of electrical connection realized by the electrical connection element 56 are distinct. Then, the electrical connection is relieved from mechanical forces thereby enhancing durability of the electrical connection.

The embodiment of the holding member 42 shown in Figure 4 is realized as a spring. The spring may comprise metal or suitable plastic material. In another embodiment the holding member may comprise elastic material like for example rubber or foam. The rubber or foam may be provided as a single elongated strip, or as multiple spaced apart separate pieces, having a square, rectangular, C-shaped or crescent-shaped cross section. Elastic material is easy to handle and may be glued to an inner wall of the battery compartment 40. The thickness of the elastic material in a direction perpendicular to the central axis 48 is sufficient to immobilize an unused battery 18 with a diameter d1 inside the battery compartment 40. The elastic material has a shape or elasticity allowing the battery 18 to expand its diameter by (at least) 5 to 10 percent.

In Figure 4 a single holding member 42 is shown. The holding member 42 is arranged in the middle or at the central portion of the battery 18 or the battery compartment 40 with regard to the length to the battery 18 or the length of the battery compartment 40 along the central axis 48. The holding member 42 may also be arranged in the middle or the central portion of the battery 18 or the battery compartment 40 with regard to a direction perpendicular to the central axis 48 as shown in Figure 2 or 3. The holding member 42 extends almost over the entire length of the battery 18. Preferably, the holding member 42 has a length of at least 25 percent of the length of the battery 18.

In Figure 5 three holding members 142 are shown. Preferably the three holding members 142 are identical. The three holding members 142 are uniformly provided in a direction along the central axis 48. In a direction along the central axis 48 one holding element 142 is located at the middle of the battery 18. The two remaining holding members 142 are located close to the end surfaces 52 of the battery 18. This distribution of the at least two holding members 142 allows for better balancing of the battery 18. In comparison to Figure 4 now three contact points between holding member 142 and battery 18 are provided, wherein the holding members 142 are equidistantly spaced apart from one another.

In Figures 2 to 5 one holding member 42 is located at a curved inner peripheral surface or wall 46 of the battery compartment 40. Alternatively, two or more holding members may be provided along surface 46. Two holding members may be arranged to push battery 18 jointly against the wall 46. For example, the two holding members and a contact point or portion between the wall 46 and the battery 18 may form an equilateral triangle.

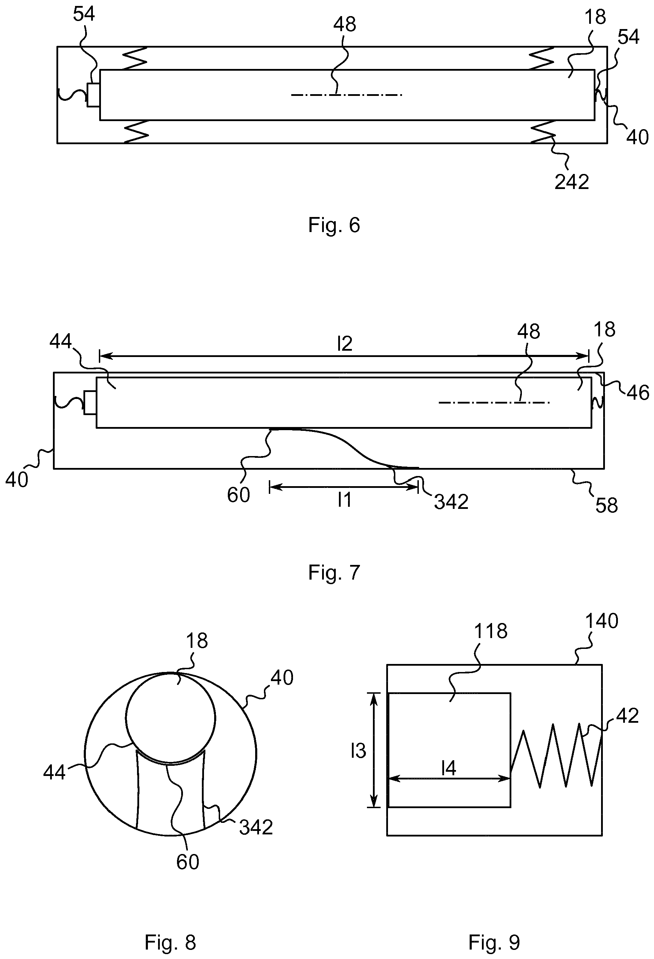

Figure 6 shows a further embodiment of holding member or members 242, which is not part of, but useful for the understanding of the claimed invention. According to this embodiment four holding members 242 are shown. Here, holding members 242 are organized in pairs opposite to the central axis 48 of the battery 18. The four holding members or two pairs of two holding members 242 are provided opposed to the central axis 48 and are adapted to hold the battery 18 in the center of the battery compartment 40. Compared to Figures 4 and 5 the embodiment of Figure 6 holds the battery 18 at the same central position inside the battery compartment 40 regardless of its diameter. This arrangement may increase stability of the electrical connection of the battery terminals 54 as their position within the battery compartment 40 is kept stable. Instead of two pairs of holding members 242 one of more than two pairs may be implemented. In addition, in this embodiment, the holding member may be provided as an annular rubber or foam sleeve that slides over the battery 18, or as separate longitudinally spaced apart annular rings placed around the battery.

The holding member 42 may be integrally formed with the battery compartment 40, for example by means of injection moulding. The holding member 42 may be attached to the battery compartment for example by means of gluing, soldering a coupling connection or the like.

The holding member 42 may be connected with the battery 18 in the same or a different manner. The battery 18 may comprise contact areas which are specially shaped e.g. flattened for improved contact with the holding member 42.

Figure 7 shows an even further embodiment of holding member 342. According to this embodiment the holding member 342 has the form of a flap which may be cut out from a wall 58 of the battery compartment 40. The wall 58 is opposite with regard to the wall 46 against which battery 18 abuts. The holding member 342 preferably has a length l1 of at least 25 percent of the length l2 of the peripheral surface 44 of the battery 18. Instead of the length of the peripheral surface 44 the length l2 could be determined as the complete length of the battery 18. The length is measured with regard to the central axis 48, i.e. parallel to the central axis 48. Alternatively, the length l1 may not designate the complete holding member 342 but only a contact portion 60 of the holding member 342 being in direct contact with the peripheral surface 44 of the battery 18.

Preferably, the flap of the holding member 342 and the wall 58 or the battery compartment 40 may be integrally formed. The flap of the holding member 342 may be cut out from the wall 58 of the battery compartment 40 thereby leaving an opening. Such implementation is easy to manufacture. Alternatively, the flap of the holding member 342 may be attached to the wall 58 so that the wall 58 is formed continuously. The holding member 342 may consist of the same material as the battery compartment 40. The material and/or the shape of the holding member 342 is adapted to exert a force directed inside the battery compartment 40. The direction of the exerted force may be perpendicular to the wall 58 of the battery compartment 40 or in other words perpendicular to the central axis 48. With a battery 18 present in the battery compartment 40 the holding member 342 exerts a force against the peripheral surface 44 of the battery 18 so that the battery 18 is immobilized against wall 46 of the battery compartment 40.

Figure 8 is a cross sectional view of Figure 7. As shown, the contact portion 60 of the holding member 342 may be adapted to the peripheral surface 44 of the battery 18. The contact portion 60 may correspond to the shape or the peripheral surface 44 of the battery 18. Such a contact portion 60 keeps the battery 18 more stable as lateral inclination of the battery 18 is prohibited. The contact portion 60 may contact the battery 18 over an area or part of the peripheral surface 44. Alternatively, contact portion 60 may contact battery 18 along at least two edges. The contact portion 60 contacts the battery 18 at least at two distinct points. The outline of the contact portion 60 may be adapted to an unused battery having a diameter d1, a used battery 118 having a diameter d2 or any diameter in between those two diameters.

While cylindrical batteries 18 with a circular cross section have been discussed it may also be possible to implement batteries having rectangular, conical or irregular shapes. The shape of the battery compartment may be adapted accordingly. The battery compartment may at least have the size and/or shape to accommodate such a battery.

In Figure 9 a cross section of a battery compartment 140 is shown. In contrast to the preceding Figures the battery compartment 140 has a rectangular shape. Inside the battery compartment 140 a rectangular rechargeable battery 118 is arranged. The depicted rechargeable battery 118 may be a used or a new battery. The battery 118 has a first length l3 perpendicular to the working direction of the holding member 42. The battery 118 has a second length l4 parallel to the working direction of the holding member 42. Both lengths or dimensions l3 and l4 may be equal or distinct.

The battery compartment 140 has a length or dimension L which is larger than the length or dimension l4 of the battery 118. In order to hold or immobilize the battery 118 inside the battery compartment 140 the holding member 42 is biased against the battery 118. The holding member 42 presses against the battery 118 so that the battery 118 is pressed against a wall of the battery compartment 140 or against another part. Such arrangement fixes or secures the battery 118 regardless of its size or its change of size. The flexible biased holding member 42 compensates for size changes of the battery 118, e.g. due to increased load cycles and keeps the battery 118 still fixed. Advantageously, undesired movement of the battery 118 is prevented. The holding member 42 has the same function as described in conjunction with Figures 2 and 3.

While holding members 42 have been depicted and discussed as being arranged completely inside the battery compartment 40 it is also possible to arrange the holding member at least partly outside the battery compartment 40. Then, the holding member is pressing against the battery through an opening of a wall of the battery compartment. In a further alternative approach the holding member may be arranged completely outside the battery compartment. In such a case the holding member presses against an outer wall of the battery compartment. The battery compartment or at least a part of a battery compartment may be flexible so that it is deformable by the holding member thereby guaranteeing indirect contact of the holding member to the battery. Alternatively, the battery compartment may have movable portion which is pressed or actuated by the holding member against the battery and thereby keeps the battery immobile.

According to the present invention a holding member 42 for a rechargeable battery 18 is introduced which immobilizes the rechargeable battery 18 inside the battery compartment 40 regardless of the diameter of the battery which may vary due to the number of experienced duty cycles. Further, the holding member 42 is even beneficial for non-removable soldered batteries. Usually, those batteries are already fixated inside a compartment or the like. The holding member 42 allows for expansion of the battery 18 while keeping it immobile and reducing stress on the soldered electrical connections.

## Claims

1. An electronic cigarette, comprising:
- a housing (12) including a battery compartment (40);
- a rechargeable battery (18) arranged inside the battery compartment (40), wherein the battery (18) comprises two end surfaces (52) opposed along a central axis (48) and a peripheral surface (44) connecting the end surfaces (52) and wherein battery terminals (54) are provided on at least one of the end surfaces (52); and
- a holding member (42) biased against the peripheral surface (44) of the battery (18) and adapted to secure the battery (18) inside the battery compartment (40) regardless of its change of size,
**characterized in that**
the holding member (42) is adapted to press the battery (18) against a wall (46) of the battery compartment (40) opposite to the holding member (42).

2. The electronic cigarette according to claim 1, wherein the holding member (42) comprises elastic material.

3. The electronic cigarette according to claim 1, wherein the holding member (42) comprises a spring.

4. The electronic cigarette according to claim 1, wherein the holding member (342) is a flap cut out from a wall (58) of the battery compartment (40).

5. The electronic cigarette according to one of claims 1 to 4, wherein the holding member (342) comprises a contact portion (60) adapted to contact the battery (18) and wherein the contact portion (60) is complementary to the peripheral surface (44) of the battery (18).

6. The electronic cigarette according to one of claims 1 to 5, wherein the length (l₁) of the holding member along the central axis (48) is at least 25% of the length (l₂) of the peripheral surface (44) of the battery (18) along the same axis.

7. The electronic cigarette according to one of claims 1 to 6, wherein the holding member (42) is arranged to contact the middle of the peripheral surface (44) of the battery (18).

8. The electronic cigarette according to one of claims 1 to 7, wherein a plurality of holding members (142) is provided spaced along the lateral extent of the battery compartment (40).

9. The electronic cigarette according to one of claims 1 to 7, wherein two holding members (242) are provided on opposite sides of the central axis (48) of the battery (18) and are adapted to hold the battery (18) in a centre of the battery compartment (40).

10. The electronic cigarette according to one of claims 1 to 9, wherein the holding member (42) is arranged inside the battery compartment (40).

11. The electronic cigarette according to one of claims 1 to 10, wherein the size (D) of the battery compartment (40) in a direction perpendicular to the central axis (48) is adapted to accommodate a battery (18) with maximal size expansion due to charge/discharge cycles.

12. The electronic cigarette according to one of claims 1 to 11, wherein the battery (18) is fixed inside the battery compartment (40).

13. The electronic cigarette according to claim 12, wherein the battery (18) is soldered at its battery terminals (54) inside the battery compartment (40).

## Patentansprüche

1. Elektronische Zigarette, umfassend:
- ein Gehäuse (12), das ein Batteriefach (40) einschließt;
- eine innerhalb des Batteriefachs (40) angeordnete wiederaufladbare Batterie (18), wobei die Batterie (18) zwei entlang einer Mittelachse (48) entgegengesetzte Endflächen (52) und eine die Endflächen (52) verbindende Umfangsfläche (44) umfasst und wobei an zumindest einer der Endflächen (52) Batterieanschlüsse (54) bereitgestellt sind; und
- ein Halteelement (42), das gegen die Umfangsfläche (44) der Batterie (18) vorgespannt und dazu angepasst ist, die Batterie (18) innerhalb des Batteriefachs (40) ungeachtet ihrer Größenänderung zu sichern,
**dadurch gekennzeichnet, dass**
das Halteelement (42) dazu angepasst ist, die Batterie (18) gegen eine dem Halteelement (42) entgegengesetzte Wand (46) des Batteriefachs (40) zu drücken.

2. Elektronische Zigarette nach Anspruch 1, wobei das Halteelement (42) elastisches Material umfasst.

3. Elektronische Zigarette nach Anspruch 1, wobei das Halteelement (42) eine Feder umfasst.

4. Elektronische Zigarette nach Anspruch 1, wobei das Halteelement (342) eine aus einer Wand (58) des Batteriefachs (40) ausgeschnittene Lasche ist.

5. Elektronische Zigarette nach einem der Ansprüche 1 bis 4, wobei das Halteelement (342) einen Kontaktabschnitt (60) umfasst, der dazu angepasst ist, mit der Batterie (18) in Kontakt zu stehen, und wobei der Kontaktabschnitt (60) zur Umfangsfläche (44) der Batterie (18) komplementär ist.

6. Elektronische Zigarette nach einem der Ansprüche 1 bis 5, wobei die Länge (l₁) des Halteelements entlang der Mittelachse (48) zumindest 25 % der Länge (l₂) der Umfangsfläche (44) der Batterie (18) entlang derselben Achse ist.

7. Elektronische Zigarette nach einem der Ansprüche 1 bis 6, wobei das Halteelement (42) dazu angeordnet ist, mit der Mitte der Umfangsfläche (44) der Batterie (18) in Kontakt zu stehen.

8. Elektronische Zigarette nach einem der Ansprüche 1 bis 7, wobei eine Mehrzahl von Halteelementen (142) entlang der seitlichen Erstreckung des Batteriefachs (40) beabstandet bereitgestellt ist.

9. Elektronische Zigarette nach einem der Ansprüche 1 bis 7, wobei zwei Halteelemente (242) an entgegengesetzten Seiten der Mittelachse (48) der Batterie (18) bereitgestellt und dazu angepasst sind, die Batterie (18) in einer Mitte des Batteriefachs (40) zu halten.

10. Elektronische Zigarette nach einem der Ansprüche 1 bis 9, wobei das Halteelement (42) innerhalb des Batteriefachs (40) angeordnet ist.

11. Elektronische Zigarette nach einem der Ansprüche 1 bis 10, wobei die Größe (D) des Batteriefachs (40) in einer zur Mittelachse (48) senkrechten Richtung dazu angepasst ist, eine Batterie (18) mit maximaler Größenexpansion aufgrund von Lade-/Entladezyklen aufzunehmen.

12. Elektronische Zigarette nach einem der Ansprüche 1 bis 11, wobei die Batterie (18) innerhalb des Batteriefachs (40) befestigt ist.

13. Elektronische Zigarette nach Anspruch 12, wobei die Batterie (18) an ihren Batterieanschlüssen (54) innerhalb des Batteriefachs (40) gelötet ist.

## Revendications

1. Cigarette électronique, comprenant :
- un boîtier (12) comprenant un compartiment de batterie (40) ;
- une batterie rechargeable (18) disposée à l'intérieur du compartiment de batterie (40), la batterie (18) comprenant deux surfaces d'extrémité (52) opposées le long d'un axe central (48) et une surface périphérique (44) reliant les surfaces d'extrémité (52) et des bornes de batterie (54) étant fournies sur au moins l'une des surfaces d'extrémité (52) ; et
- un membre de maintien (42) poussé contre la surface périphérique (44) de la batterie (18) et adapté pour bloquer la batterie (18) à l'intérieur du compartiment de batterie (40) indépendamment de son changement de taille,
**caractérisée en ce que**
le membre de maintien (42) est adapté pour presser la batterie (18) contre une paroi (46) du compartiment de batterie (40) opposée au membre de maintien (42).

2. Cigarette électronique selon la revendication 1, le membre de maintien (42) comprenant un matériau élastique.

3. Cigarette électronique selon la revendication 1, le membre de maintien (42) comprenant un ressort.

4. Cigarette électronique selon la revendication 1, le membre de maintien (342) étant un volet découpé dans une paroi (58) du compartiment de batterie (40).

5. Cigarette électronique selon l'une des revendications 1 à 4, le membre de maintien (342) comprenant une portion de contact (60) adaptée pour faire contact avec la batterie (18) et la portion de contact (60) étant complémentaire de la surface périphérique (44) de la batterie (18).

6. Cigarette électronique selon l'une des revendications 1 à 5, la longueur (l₁) du membre de maintien (11) le long de l'axe central (48) étant d'au moins 25 % de la longueur (l₂) de la surface périphérique (44) de la batterie (18) le long du même axe.

7. Cigarette électronique selon l'une des revendications 1 à 6, le membre de maintien (42) étant disposé pour être en contact avec le milieu de la surface périphérique (44) de la batterie (18).

8. Cigarette électronique selon l'une des revendications 1 à 7, une pluralité de membres de maintien (142) étant fournis espacés le long de l'extension latérale du compartiment de batterie (40).

9. Cigarette électronique selon l'une des revendications 1 à 7, deux membres de maintien (242) étant fournis sur des côtés opposés de l'axe central (48) de la batterie (18) et étant adaptés pour maintenir la batterie (18) dans un centre du compartiment de batterie (40).

10. Cigarette électronique selon l'une des revendications 1 à 9, le membre de maintien (42) étant disposé à l'intérieur du compartiment de batterie (40).

11. Cigarette électronique selon l'une des revendications 1 à 10, la taille (D) du compartiment de batterie (40) dans une direction perpendiculaire à l'axe central (48) étant adaptée pour accueillir une batterie (18) avec une dilatation de taille maximale du fait des cycles de charge/décharge.

12. Cigarette électronique selon l'une des revendications 1 à 11, la batterie (18) étant fixée à l'intérieur du compartiment de batterie (40).

13. Cigarette électronique selon la revendication 12, la batterie (18) étant soudée à ses bornes de batterie (54) à l'intérieur du compartiment de batterie (40).
